# EUROPÄISCHE PATENTANMELDUNG

(11) **EP 4 039 187 A1**
(43) Veröffentlichungstag der Anmeldung: **10.08.2022**
(21) Anmeldenummer: 21155565.1
(22) Anmeldetag: 05.02.2021
(51) Int. Cl.: A61B 5/08, A61B 5/00, G06N 3/02, G06N 5/02, G10L 17/26, G10L 15/16, G10L 25/66

(54) **COMPUTERIMPLEMENTIERTES VERFAHREN UND WERKZEUG SOWIE DATENVERARBEITUNGSGERÄT ZUM ERKENNEN VON OBEREN ATEMWEGSERKRANKUNGEN BEIM MENSCHEN**

(71) Anmelder: Siemens Aktiengesellschaft, 80333 München (DE)
(72) Erfinder: Hahn, Christian, 91315 Höchstadt a.d. Aisch (DE)

(57) **Zusammenfassung**

Um obere Atemwegserkrankungen beim Menschen anzugeben, bei dem die Erkrankung kontaktlos, nicht-invasiv zu erkennen ist und keinen Arztbesuch erfordert, wird es vorgeschlagen, eine die menschliche Stimme einer Testperson charakterisierenden Kenngröße (KG) zu generieren, indem
- ein einen modulierten Schall durch Umwandlung erzeugtes Audiosignal (AS) mit einer den modulierten Schall repräsentierenden Modulationsspannung erfasst wird,
- ein Digitalsignal (DS) aus digital, zeitintervall-basiert erfassten Werten der Modulationsspannung erzeugt wird,
- das Digitalsignal (DS) in, vorzugsweise überlappende, Digitalsignalabschnitte (DSA) definierter Länge - sogenannter "frames"- zerlegt wird,
- die Digitalsignalabschnitte (DSA) bzw. "frames" zur Gewinnung von Stimmattributen (STA) als Extraktion von Merkmalen der Stimme zwecks deren Klassifizierung analysiert werden,
- ein Merkmalsvektor (MV) auf der Basis einer Zeitreihenanalyse der extrahierten Merkmale bestimmt wird, und
die Kenngröße (KG) in einen auf die Klassifikation von schallumgewandelten Audiosignalen trainierten, tiefen neuronalen Netzwerk-Regressor (NWR) zum Ausgeben eines Wahrscheinlichkeitswertes (WKW) einzugeben, der angibt mit welcher Wahrscheinlichkeit die Testperson an den oberen Atemwegen erkrankt ist.

## Beschreibung

Die Erfindung betrifft ein computerimplementiertes Verfahren und Werkzeug sowie ein Datenverarbeitungsgerät zum Erkennen von oberen Atemwegserkrankungen beim Menschen.

Zur Erkennung Erkrankungen von oberen Atemwegen beim Menschen, die z.B. durch einen Coronavirus SARS-CoV-2 <COVID-19> hervorgerufen werden, kommen aktuell, nach derzeitigem Stand der Technik folgende Methoden zum Einsatz:
1. Eine erste labordiagnostische Methode erfolgt durch direkten Erregernachweis, so z.B. durch einen PCR-Test ("Polymerase Chain Reaction <PCR>"). Mit einem solchen Test lassen sich die betreffenden Erreger bereits kurz nach der Ansteckung bei Menschen mit und ohne spezifischen Symptomen nachweisen. In der Regel sind hierfür Abstriche aus dem Bereich der oberen Atemwege (Nase, Rachen) erforderlich. Fehler bei einer Abnahme eines Abstrichs und Versand der Proben können die Qualität der Ergebnisse negativ beeinflussen, insbesondere können hierdurch falsch-negative Ergebnisse hervorgerufen werden.
2. Eine erste labordiagnostische Methode erfolgt durch Antikörpernachweise. Mit diesen Antikörpernachweis kann nachgewiesen werden, dass ein Mensch mit einem bestimmten Erreger infiziert ist oder infiziert war. Der Nachweis ist erst erfolgreich, wenn der Körper spezifische Antikörper gebildet hat - also typischerweise erst mehrere Tage nach Erkrankungsbeginn.
3. Eine dritte Methode erfolgt durch Kontakt-Tracing, bei dem mögliche Folge-Fälle eines bestätigten Falles ermittelt werden. Das Tracing kann manuell oder durch den Einsatz von Tracing-Apps erfolgen, wie z.B. auf Basis eines offenen Full-Stack-Protokolls PEPP-PT (Pan-European Privacy-Preserving Proximity Tracing), das die digitale Kontaktverfolgung infizierter Teilnehmer erleichtern soll.
4. Eine vierte Methode erfolgt durch die Analyse eines Körpertemperatur-Verlaufs, das als experimentelles Verfahren erprobt wird. Es erfordert das permanente Tragen eines Temperaturmessers, z.B. Laufuhren, Smartwatches etc., - sogenannte "Wearables" - und in der Regel die Übertragung der Daten auf einen Server, der die Berechnungen durchführt, da Rechenleistung und Speicherkapazität der "Wearables" im Moment nicht ausreichend sind.

Die der Erfindung zugrundeliegende Aufgabe besteht darin, ein Computer-implementiertes Verfahren und Werkzeug sowie ein Datenverarbeitungsgerät zum Erkennen von oberen Atemwegserkrankungen beim Menschen anzugeben, beidem die Erkrankung kontaktlos, nicht-invasiv zu erkennen ist und keinen Arztbesuch erfordert.

Die vorstehend bezeichnete Aufgabe wird durch das im Patentanspruch 1 angegebene computerimplementierte Verfahren gelöst.

Weiterhin wird diese Aufgabe durch das im Patentanspruch 7 angegebene computerimplementierte Werkzeug gelöst.

Darüber hinaus wird diese Aufgabe durch das im Patentanspruch 13 angegebene Datenverarbeitungsgerät gelöst. sowie ein

Die der Erfindung zugrundeliegende Idee gemäß der in den Ansprüchen 1, 7 und 13 jeweils angegebenen technischen Lehre besteht darin,
eine die menschliche Stimme einer Testperson charakterisierenden Kenngröße zu generieren, indem
- ein einen modulierten Schall durch Umwandlung erzeugtes Audiosignal mit einer den modulierten Schall repräsentierenden Modulationsspannung erfasst wird,
- ein Digitalsignal aus digital, zeitintervall-basiert erfassten Werten der Modulationsspannung erzeugt wird,
- das Digitalsignal in, vorzugsweise überlappende, Digitalsignalabschnitte definierter Länge - sogenannter "frames"-zerlegt wird,
- die Digitalsignalabschnitte bzw. "frames" zur Gewinnung von Stimmattributen als Extraktion von Merkmalen der Stimme zwecks deren Klassifizierung analysiert werden,
- ein Merkmalsvektor auf der Basis einer Zeitreihenanalyse der extrahierten Merkmale bestimmt wird, und
die Kenngröße in einen auf die Klassifikation von schallumgewandelten Audiosignalen trainierten, tiefen neuronalen Netzwerk-Regressor zum Ausgeben eines Wahrscheinlichkeitswertes einzugeben, der angibt mit welcher Wahrscheinlichkeit die Testperson an den oberen Atemwegen erkrankt ist.

Die vorgeschlagene Lösung zeichnet sich insbesondere dadurch aus, dass mögliche Erkrankungen der oberen Atemwege, die z.B. durch einen Coronavirus SARS-CoV-2 <COVID-19> hervorgerufen werden (vgl. Ansprüche 6 und 12), beim Menschen ohne spezifische Symptome, also wenn die Erkrankung asymptomatisch ist oder präsymptomatisch erkannt werden können (vgl. Ansprüche 5 und 11). Zudem erfordert die vorgeschlagene Lösung keine Spezial-Hardware und kann als Software-Lösung auf in der Bevölkerung weit verbreiteten Geräten, wie z.B. Smartphones, Tablets oder Notebooks realisiert werden. Eine Weitergabe von Daten - insbesondere für eine zentrale Verarbeitung oder Speicherung - ist bei der vorgeschlagenen Lösung nicht erforderlich.

Weiterhin sind bei der vorgeschlagenen
- lediglich Sprach-Samples der zu testenden Person erforderlich und keine Abstriche oder Blutproben,
- die Aufnahme der Sprach-Samples erfolgt vorzugsweise über ein Mikrophon,
- die vorgeschlagene Lösung kann von jedem selbst durchgeführt werden, es ist also kein Arztbesuch erforderlich
- die vorgeschlagene Lösung ist eine Software-Only-Lösung und kann auf Standard-Hardware, z.B. ein Smartphone, ein Tablet oder ein Notebook, realisiert werden, so dass die Anschaffung einer teuren Spezial-Hardware nicht erforderlich ist.
- bei der vorgeschlagenen Lösung ist das Ergebnis sofort verfügbar, so steht das Ergebnis wenige Sekunden nach der Aufnahme der Samples zur Verfügung,
- die vorgeschlagene Lösung wahrt die Privatsphäre, da die zu testende Person die vorgeschlagene Lösung selbst anwenden kann und keinerlei Proben oder Daten an Dritte weitergegeben werden.

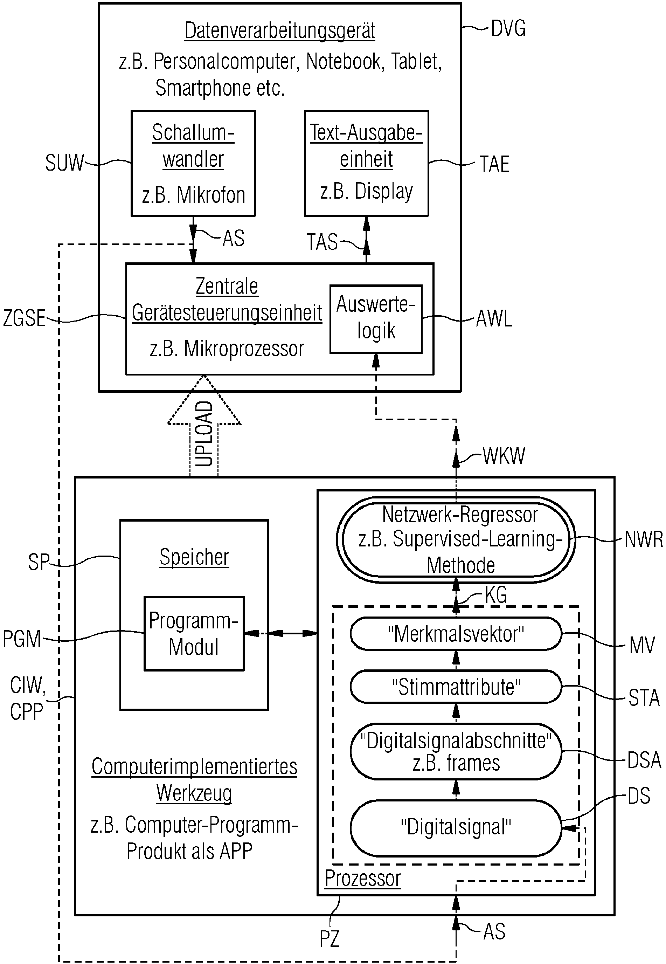

Weitere Vorteile der Erfindung ergeben sich aus den Unteransprüchen sowie der nachfolgenden Beschreibung eines Ausführungsbeispiels der Erfindung anhand der FIGUR erläutert. Diese zeigt
ein Szenario zum Erkennen von oberen Atemwegserkrankungen beim Menschen

Zentrale Komponente gemäß diesem in der FIGUR dargestellten Szenario zum Erkennen der oberen Atemwegserkrankungen beim Menschen, die z.B. durch einen Coronavirus SARS-CoV-2 <COVID-19> hervorgerufen werden können, ist ein computerimplementiertes Werkzeug CIW, in dem ein zu dem genannten Zweck ablaufendes computerimplementiertes Verfahren abläuft, das vorzugsweise ein Computer-Programm-Produkt CPP ist und zudem als eine Anwendungssoftware - auch als "APP" bezeichnet - ausgebildet ist.

Darüber hinaus ist dieses computerimplementierte Werkzeug CIW bzw. das Computer-Programm-Produkt CPP zum Erkennen der oberen Atemwegserkrankungen beim Menschen als "Upload" in ein Datenverarbeitungsgerät DVG hochladbar. Diese Aktion oder dieser Zustand ist in der FIGUR durch den gestrichelten Doppelpfeil (Pfeil mit doppelter Basis) dargestellt.

Das Datenverarbeitungsgerät DVG kann dabei beispiels- und vorzugsweise ein Personal Computer, Notebook, Tablet oder Smartphone, etc. sein, mit dem nach dem Hochladen des als APP ausgebildeten computerimplementierten Werkzeugs CIW bzw. des Computer-Programm-Produkts CPP die Erkrankungen der oberen Atemwege beim Menschen erkannt werden können.

Zentrale Komponente des Datenverarbeitungsgeräts DVG - nicht zuletzt für die Erkennung der oberen Atemwegserkrankungen, sondern auch ganz generell für geräteinterne Funktions- und Steuerungsabläufe - ist eine Zentrale Gerätesteuerungseinheit ZGSE, die in bekannter Weise z.B. als Mikroprozessor ausgebildet ist. Neben dieser Zentralen Gerätesteuerungseinheit ZGSE weist das Datenverarbeitungsgerät DVG eine Vielzahl weiterer Komponenten auf. Für die Erkennung der oberen Atemwegserkrankungen beim Menschen spielen aber nur - wie in der FIGUR dargestellt - eine Text-Ausgabeeinheit TAE und ein Schallumwandler SUW eine Rolle. Beide, die Text-Ausgabeeinheit TAE und der Schallumwandler SUW, sind u.a. für die Erkennung der oberen Atemwegserkrankungen mit der Zentralen Gerätesteuerungseinheit ZGSE verbunden. Während die Text-Ausgabeeinheit TAE z.B. und vorzugsweise als Display oder Bildschirm ausgebildet ist, ist der Schallumwandler SUW beispielsweise als Mikrofon ausgebildet.

Die Funktions- und Wirkungsweise des Schallumwandlers SUW bzw. des Mikrofons besteht bekanntlich darin, dass ein durch die Stimme eines Menschen erzeugter, entsprechend modulierter Schall in ein Audiosignal AS mit einer den modulierten Schall repräsentierenden Modulationsspannung umgewandelt wird.

Bei der Text-Ausgabeeinheit TAE bzw. dem Displays besteht die Funktions- und Wirkungsweise zudem bekanntlich darin, dass ein Text mit einer darin enthaltenen Aussage, also eine Text-Aussage TAS, ausgeben und dargestellt werden kann. Um beispielsweise aus einem beliebig wählbaren Datum, z.B. einem Zahlenwert, eine Text-Aussage TAS generieren zu können, bedarf es einer bestimmten Logik, die dieses gewährleistet. Im vorliegenden Fall zur Erkennung der oberen Atemwegserkrankungen beim Menschen, wie es nachfolgend weiter ausgeführt werden wird, ist eine Auswertelogik AWL zur Datum-Text-Konvertierung erforderlich, die ein Datum, z.B. einen Zahlenwert, in eine das Datum bzw. den Zahlenwert entsprechende, adäquate Text-Aussage TAS konvertiert. Diese Auswertelogik AWL ist zu diesem Zweck vor dem Hintergrund der Erkennung der oberen Atemwegserkrankungen beim Menschen in der Zentralen Gerätesteuerungseinheit ZGSE des Datenverarbeitungsgeräts DVG enthalten ist.

Jetzt aber zu dem computerimplementierten Werkzeug CIW bzw. dem Computer-Programm-Produkt CPP als die zentrale Komponente gemäß dem in der FIGUR dargestellten Szenario zum Erkennen der oberen Atemwegserkrankungen beim Menschen.

Zu diesem Zweck weist das computerimplementierte Werkzeug CIW bzw. das Computer-Programm-Produkt CPP einen nicht-flüchtigen, lesbaren Speicher SP, in dem prozessorlesbare Steuerprogrammbefehle eines Programm-Moduls PGM zum Erkennen der oberen Atemwegserkrankungen gespeichert sind, und einen mit dem Speicher SP verbundenen Prozessor PZ auf, der die Steuerprogrammbefehle des Programm-Moduls PGM zum Erkennen der oberen Atemwegserkrankungen ausführt.

Um diese Ziel zu erreichen, sind das Programm-Modul PGM derart beschaffen und der die Steuerprogrammbefehle des Programm-Moduls PGM zur Erkennung der oberen Atemwegserkrankungen ausführende Prozessor PZ derart ausgebildet, dass eine die menschliche Stimme einer Testperson charakterisierenden Kenngröße KG generiert wird und diese Kenngröße KG einem auf die Klassifikation von schallumgewandelten Audiosignalen trainierten, tiefen neuronalen Netzwerk-Regressor NWR zum Erzeugen eines Wahrscheinlichkeitswertes WKW zugeführt wird (mit anderen Worten, indem die Kenngröße KG in den Netzwerk-Regressor NWR eingegeben wird), wobei durch den erzeugten Wahrscheinlichkeitswert WKW angegeben wird, mit welcher Wahrscheinlichkeit die Testperson an den oberen Atemwegen erkrankt ist.

Der Netzwerk-Regressor NWR wird dabei vorzugsweise nach Maßgabe einer "Supervised Learning"-Methode trainiert, um z.B. Klassifikationsaufgabe zu erfüllen.

Der Wahrscheinlichkeitswert WKW wird zur Konvertierung in die adäquate Text-Aussage TAS an die Auswertelogik AWL in der Zentralen Gerätesteuerungseinheit ZGSE des Datenverarbeitungsgeräts DVG übermittelt. Dies kann gemäß der FIGUR und aufgrund der vorstehenden Beschreibung dieser FIGUR z.B. dadurch erfolgen, dass das computerimplementierte Werkzeug CIW bzw. das Computer-Programm-Produkt CPP in die Zentralen Gerätesteuerungseinheit ZGSE geladen wird und dort als in die Zentrale Gerätesteuerungseinheit ZGSE ladbare APP die Erkennung der oberen Atemwegserkrankungen ermöglicht.

Die Auswertelogik AWL wandelt den Wahrscheinlichkeitswert WKW in die einfach verstehbare Text-Aussage TAS um, so z.B. in die Text-Aussage TAS "wahrscheinlich gesund" oder in die Text-Aussage TAS "wahrscheinlich erkrankt" oder in die Text-Aussage TAS "möglicherweise erkrankt".

Auf diese Art und Weise kann die Erkrankung der oberen Atemwege sowohl präsymptomatisch als auch asymptomatisch erkannt werden.

Weshalb soll zu dem angegeben Zweck die Kenngröße KG erzeugt werden und Was ist diese Kenngröße KG?

Zu dem "Weshalb": Menschen können anhand des Klangs der Sprache eines anderen Menschen wahrnehmen, dass dieser erkältet ist oder eine Atemwegserkrankung durchmacht. Oft hört man bereits, dass bei einem anderen Menschen eine Erkältung "im Anzug" ist - sich ankündigt durch zeitliches Näherkommen, bevor dieser selbst erste spezifische Symptome, z.B. Halsschmerzen, wahrnimmt. Möglicherweise handelt es sich um einen Selbstschutzmechanismus des Menschen, der sich im Zuge der Evolution ausgebildet hat.

Aus Sicht der Spracherzeugung ist diese Wahrnehmung durchaus plausibel erklärbar: "Ein periodisches Anregungssignal (Stimmbänder) wird durch einen 'linearen Filter' (Mund, Zunge, Nasenhöhlen, ...) geformt" [1].

Erkrankungen der oberen Atemwege spielen sich gerade in diesen Bereichen ab. Sie verursachen dort Entzündungen oder Schwellungen. Auch wenn diese im prä- oder asymptomatischen Fall nur leicht ausfallen, können sie (zumindest unterschwellig) wahrnehmbare Auswirkungen auf die Stimme eines Menschen haben.

Durch das Generieren der Kenngröße KG und dem Zuführen der Kenngröße KG zu dem Netzwerk-Regressor NWR zum Erzeugen des Wahrscheinlichkeitswertes WKW wird diese Wahrnehmungsfähigkeit des menschlichen Geistes unter Verwendung künstlicher Intelligenz nachgebildet.

Zu dem "Was": Die Kenngröße KG wird von dem das Programm-Modul PGM zur Erkennung der oberen Atemwegserkrankungen ausführenden Prozessor PZ generiert, indem zunächst das den modulierten Schall durch Umwandlung erzeugte Audiosignal AS mit der den modulierten Schall repräsentierenden Modulationsspannung erfasst wird und dem Prozessor PZ zuführbar ist. Auch dies kann wieder gemäß der FIGUR und aufgrund der vorstehenden Beschreibung dieser FIGUR z.B. dadurch erfolgen, dass das computerimplementierte Werkzeug CIW bzw. das Computer-Programm-Produkt CPP in die Zentralen Gerätesteuerungseinheit ZGSE geladen wird und dort als in die Zentrale Gerätesteuerungseinheit ZGSE ladbare APP die Erkennung der oberen Atemwegserkrankungen ermöglicht.

Für die Generierung der Kenngröße KG ist es zudem erforderlich, dass ein Digitalsignal DS aus digital, zeitintervall-basiert erfassten Werten der Modulationsspannung erzeugt wird, also ein sogenanntes "Sampling" durchgeführt wird. Beim "Sampling" werden die erfassten Signale digitalisiert. Sampling-Rate und Datenformat müssen auf noch weitere Verarbeitungsschritte für die Kenngröße-Generierung abgestimmt sein, um ein optimales Ergebnis zu gewährleisten.

Als weitere Verarbeitungsschritte für die Kenngröße-Generierung wird benötigt, dass
- das Digitalsignal DS in Digitalsignalabschnitte DSA definierter Länge - sogenannter "frames" - zerlegt wird, die sich z.B. überlappen können,
- die Digitalsignalabschnitte DSA bzw. "frames" zur Gewinnung von Stimmattributen oder Stimmeigenschaften STA als Extraktion von Merkmalen der Stimme zwecks deren Klassifizierung analysiert werden und
- ein Merkmalsvektor MV auf der Basis einer Zeitreihenanalyse der extrahierten Merkmale bestimmt wird.

Die Extraktion der Merkmalen der Stimme zwecks deren Klassifizierung erfolgt ähnlich wie die "Feature Extraction" bei der Emotionserkennung (vgl. [2]), z.B. standardgemäß zur Spezifikation ETSI ES 202 211 V1.1.1 (vgl. [3]).

Der Merkmalsvektor MV wird dem Netzwerk-Regressor NWR als Input zugeführt und entspricht somit der Kenngröße KG. Der Netzwerk-Regressor NWR ist auf die Klassifikation des Sprachsignals trainiert. Der Output des Netzwerk-Regressors NWR, der durch den ausgegebenen Wahrscheinlichkeitswert WKW definiert ist, gibt an, mit welcher Wahrscheinlichkeit die Testperson "erkrankt" oder "gesund" ist.

### Referenzen

[1] "Wikipedia. Die freie Enzyklopädie," Wikimedia Foundation Inc., 30 05 2020. [Online]. Available: https://de.wikipedia.org/wiki/Mel_Frequency_Cepstral_Coeffici ents. [Zugriff am 09 10 2020].
[2] S. Casale, A. Russo, G. Scebba und S. Serrano, "Speech Emotion Classification using Machine Learning Algorithms," in The IEEE International Conference on Semantic Computing, 2008.
[3] ETSI ES 202 211 V1.1.1 - speech processing, transmission and quality aspects (stq); distributed speed recognition; extended front-end feature extraction algorithm; compression algorithms; back-end speech recognition algorithm, European Telecommunications Standards Institute, 2003.

## Patentansprüche

1. Computerimplementiertes Verfahren zum Erkennen von oberen Atemwegserkrankungen beim Menschen,
**gekennzeichnet durch:**
**a)** Generieren einer die menschliche Stimme einer Testperson charakterisierenden Kenngröße (KG) durch
**a1**) Erfassen eines einen modulierten Schall durch Umwandlung erzeugten Audiosignals (AS) mit einer den modulierten Schall repräsentierenden Modulationsspannung,
**a2)** Erzeugen eines Digitalsignals (DS) aus digital, zeitintervall-basiert erfasste Werten der Modulationsspannung,
**a3)** Zerlegen des Digitalsignals (DS) in, vorzugsweise überlappende, Digitalsignalabschnitte (DSA) definierter Länge - sogenannter "frames",
**a4)** Analysieren der Digitalsignalabschnitte (DSA) bzw. "frames" zur Gewinnung von Stimmattributen (STA) als Extraktion von Merkmalen der Stimme zwecks deren Klassifizierung,
**a5)** Bestimmen eines Merkmalsvektors (MV) auf der Basis einer Zeitreihenanalyse der extrahierten Merkmale,
**b)** Eingeben der Kenngröße (KG) in einen auf die Klassifikation von schallumgewandelten Audiosignalen trainierten, tiefen neuronalen Netzwerk-Regressor (NWR) zum Ausgeben eines Wahrscheinlichkeitswertes (WKW), der angibt mit welcher Wahrscheinlichkeit die Testperson an den oberen Atemwegen erkrankt ist.

2. Computerimplementiertes Verfahren nach Anspruch 1,
**dadurch gekennzeichnet, dass**
der Wahrscheinlichkeitswert (WKW) in eine adäquate Text-Aussage (TAS) konvertiert wird.

3. Computerimplementiertes Verfahren nach Anspruch 1 oder 2,
**dadurch gekennzeichnet, dass**
die Merkmalsextraktion im Wesentlichen in Analogie zu einer Emotionserkennung beim Menschen, z.B. standardgemäß zur Spezifikation ETSI ES 202 211 V1.1.1, durchgeführt wird.

4. Computerimplementiertes Verfahren nach einem der Ansprüche 1 bis 3, **dadurch gekennzeichnet, dass** der Netzwerk-Regressor (NWR) nach Maßgabe einer "Supervised Learning"-Methode trainiert wird.

5. Computerimplementiertes Verfahren nach einem der Ansprüche 1 bis 4, **dadurch gekennzeichnet, dass** die Erkrankung der oberen Atemwege präsymptomatisch oder asymptomatisch erkannt wird.

6. Computerimplementiertes Verfahren nach einem der Ansprüche 1 bis 5, **dadurch gekennzeichnet, dass** die Erkrankung der oberen Atemwegserkrankungen durch einen Coronavirus SARS-CoV-2 <COVID-19> hervorgerufen wird.

7. Computerimplementiertes Werkzeug (CIW), insbesondere ein als APP ausgebildetes Computer-Programm-Produkt (CPP), zum Erkennen von oberen Atemwegserkrankungen beim Menschen, mit einem nicht-flüchtigen, lesbaren Speicher (SP), in dem prozessorlesbare Steuerprogrammbefehle eines Programm-Moduls (PGM) zum Erkennen von oberen Atemwegserkrankungen gespeichert sind, und einem mit dem Speicher (SP) verbundenen Prozessor (PZ), der die Steuerprogrammbefehle des Programm-Moduls (PGM) zum Erkennen der oberen Atemwegserkrankungen ausführt,
**dadurch gekennzeichnet, dass**
das Programm-Modul (PGM) derart beschaffen und der die Steuerprogrammbefehle des Programm-Moduls (PGM) zur Erkennung der oberen Atemwegserkrankungen ausführende Prozessor (PZ) derart ausgebildet sind, dass
**a)** einer die menschliche Stimme einer Testperson charakterisierenden Kenngröße (KG) generiert wird, indem
**a1)** ein einen modulierten Schall durch Umwandlung erzeugtes Audiosignal (AS) mit einer den modulierten Schall repräsentierenden Modulationsspannung erfasst wird und dem Prozessor (PZ) zuführbar ist,
**a2)** ein Digitalsignal (DS) aus digital, zeitintervall-basiert erfassten Werten der Modulationsspannung erzeugt wird,
**a3)** das Digitalsignal (DS) in, vorzugsweise überlappende, Digitalsignalabschnitte (DSA) definierter Länge - sogenannter "frames" - zerlegt wird,
**a4)** die Digitalsignalabschnitte (DSA) bzw. "frames" zur Gewinnung von Stimmattributen (STA) als Extraktion von Merkmalen der Stimme zwecks deren Klassifizierung analysiert werden,
**a5)** ein Merkmalsvektor (MV) auf der Basis einer Zeitreihenanalyse der extrahierten Merkmale bestimmt wird,
**b)** die Kenngröße (KG) einem auf die Klassifikation von schallumgewandelten Audiosignalen trainierten, tiefen neuronalen Netzwerk-Regressor (NWR) zum Erzeugen eines Wahrscheinlichkeitswertes (WKW) zugeführt wird, wobei durch den erzeugten Wahrscheinlichkeitswert (WKW) angegeben wird, mit welcher Wahrscheinlichkeit die Testperson an den oberen Atemwegen erkrankt ist.

8. Computerimplementiertes Werkzeug (CIW) nach Anspruch 7,
**dadurch gekennzeichnet, dass**
der Prozessor (PZ) und das Programm-Modul (PGM) zur Erkennung der oberen Atemwegserkrankungen derart ausgebildet sind, dass der Wahrscheinlichkeitswert (WKW) zur Konvertierung in eine adäquate Text-Aussage (TAS) an eine Auswertelogik (AWL) eines Datenverarbeitungsgeräts (DVG) übermittelt wird.

9. Computerimplementiertes Werkzeug (CIW) nach Anspruch 7 oder 8, **dadurch gekennzeichnet, dass** der Prozessor (PZ) und das Programm-Modul (PGM) zur Erkennung der oberen Atemwegserkrankungen derart ausgebildet sind, dass die Merkmalsextraktion im Wesentlichen in Analogie zu einer Emotionserkennung beim Menschen, z.B. standardgemäß zur Spezifikation ETSI ES 202 211 V1.1.1, durchgeführt wird.

10. Computerimplementiertes Werkzeug (CIW) nach einem der Ansprüche 7 bis 9, **dadurch gekennzeichnet, dass** der Prozessor (PZ) und das Programm-Modul (PGM) zur Erkennung der oberen Atemwegserkrankungen derart ausgebildet sind, dass der Netzwerk-Regressor (NWR) nach Maßgabe einer "Supervised Learning"-Methode trainiert wird.

11. Computerimplementiertes Werkzeug (CIW) nach einem der Ansprüche 7 bis 10, **dadurch gekennzeichnet, dass** der Prozessor (PZ) und das Programm-Modul (PGM) zur Erkennung der oberen Atemwegserkrankungen derart ausgebildet sind, dass die Erkrankung der oberen Atemwege präsymptomatisch oder asymptomatisch erkennbar ist.

12. Computerimplementiertes Werkzeug (CIW) nach einem der Ansprüche 7 bis 11, **dadurch gekennzeichnet, dass** die Erkrankung der oberen Atemwegserkrankungen durch einen Coronavirus SARS-CoV-2 <COVID-19> hervorgerufen wird.

13. Datenverarbeitungsgerät (DVG), insbesondere ein Personal Computer, Notebook, Tablet oder Smartphone, etc., zum Erkennen von oberen Atemwegserkrankungen beim Menschen mit
**i)** einer Zentralen Gerätesteuerungseinheit (ZGSE), die eine Auswertelogik (AWL) zur Datum-Text-Konvertierung enthält,
**ii)** einer Text-Ausgabeeinheit (TAE) und
**iii)** einem Schallumwandler (SUW), wobei die Text-Ausgabeeinheit (TAE) der Schallumwandler (SUW) mit der Zentralen Gerätesteuerungseinheit (ZGSE) verbunden sind, **gekennzeichnet durch**
ein computerimplementiertes Werkzeug (CIW) nach einem der Anspruche 7 bis 12, das zur Durchführung des Verfahrens nach einem der Ansprüche 1 bis 6 in die Zentrale Gerätesteuerungseinheit (ZGSE) ladbar ist.
